Europäisches Patentamt

European Patent Office

Office européen des brevets

(19)

(11) **EP 0 712 857 B1**

(12) **EUROPÄISCHE PATENTSCHRIFT**

(45) Veröffentlichungstag und Bekanntmachung des Hinweises auf die Patenterteilung:
**27.05.1998 Patentblatt 1998/22**

(51) Int Cl.[6]: **C07H 15/04**

(21) Anmeldenummer: **95117385.5**

(22) Anmeldetag: **04.11.1995**

(54) **Verfahren zur Herstellung von Alkylglycosiden**

Process for the preparation of alkylglycosides

Procédé pour la préparation d'alkylglycosides

(84) Benannte Vertragsstaaten:
**BE CH DE ES FR GB IT LI NL**

(30) Priorität: **18.11.1994 DE 4441072**

(43) Veröffentlichungstag der Anmeldung:
**22.05.1996 Patentblatt 1996/21**

(73) Patentinhaber: **Th. Goldschmidt AG**
**45127 Essen (DE)**

(72) Erfinder:
• **Grüning, Burghard, Dr.**
**D-45134 Essen (DE)**
• **Peter, Siegfried, Prof. Dr.**
**D-91080 Uttenreuth-Weiher (DE)**
• **Recksik, Manfred**
**D-45326 Essen (DE)**
• **Weidner, Eckhard, Dr.**
**D-91056 Erlangen (DE)**
• **Zhang, Zhengfeng, Dr.**
**D-91054 Erlangen (DE)**

(56) Entgegenhaltungen:
**EP-A- 0 255 331          EP-A- 0 404 226**
**EP-A- 0 639 551**

• **DATABASE WPI Week 9213 Derwent Publications Ltd., London, GB; AN 92-102825 XP002007147 & JP-A-04 049 297 (KAO CORP.), 18.Februar 1992**

**Beschreibung**

Die Erfindung betrifft ein Verfahren zur Herstellung von Alkylglycosiden, in der Literatur auch häufig als Alkylpolyglycoside bezeichnet, durch Acetalisierung von Zuckern, insbesondere Glucose, mit Alkoholen im Überschuß und nachfolgende Abtrennung des überschüssigen Alkohols durch Extraktion.

Aus der DE-OS 38 33 780 ist ein Verfahren zur direkten Herstellung von Alkylglycosiden durch Acetalisierungsreaktion von höheren primären Alkoholen mit Glycosen, insbesondere Glucose, bekannt, bei dem (in abgekürzter Zitierung)

a) Mischungen von aliphatischem, primärem Alkohol, Glycose und saurem Katalysator bei erhöhter Temperatur umgesetzt werden,
b) ein Molverhältnis Glycose zu aliphatischem Alkohol 1 : 2 bis 1 : 10 eingehalten wird,
c) das Reaktionsgemisch solange bei der erhöhten Temperatur und Unterdruck belassen wird, bis das Reaktionswasser vollständig entfernt ist,
d) das Reaktionsgemisch auf 90 °C gekühlt und eine Base zugesetzt wird, bis über die Neutralisation des Katalysators hinaus ein pH-Wert von wenigstens 8 erreicht ist und wobei man dann Normaldruck herstellt,
e) der überschüssige Alkohol auf einen Wert von unterhalb 5 Gew.-% schonend abdestilliert und
f) Wasser zur Bildung einer Paste hinzugefügt und das Produkt bei pH 8 bis 10 mit aktivem Sauerstoff gebleicht wird.

Die DE-OS 39 32 173 betrifft ein Verfahren zum destillativen Abtrennen von Alkoholen mit Kettenlängen bis 30, insbesondere zwischen 8 und 18 Kohlenstoffatomen, aus einem Gemisch von Alkylglycosiden und bei der Herstellung dieser Alkylglycoside nicht umgesetzten Alkoholen, wobei die Alkohole in zwei Stufen abgetrennt werden, mit dem Kennzeichen, daß in der ersten Stufe ein Fallfilmverdampfer und in der zweiten Stufe ein Dünnschichtverdampfer eingesetzt werden.

Mit der destillativen Entfernung des Alkohols ist eine erhebliche thermische Belastung verbunden, die die Gefahr einer Dunkelfärbung des Produktes mit sich bringt. Dies geht z. B. im Falle des Stearylglucosides soweit, daß die Entfernung von Stearylalkohol unter technischen Bedingungen praktisch nicht möglich ist, ohne das Stearylglucosid in seiner Qualität erheblich zu schädigen. Es sind aus diesem Grunde verschiedene Verfahren zur Bleichung von Alkylglycosiden beschrieben worden. Als Beispiele seien die EP-OS 0 526 710, die DE-OS 40 19 175 und die DE-OS 39 40 827 genannt, in denen die Bleichung durch Bestrahlung, durch Zusatz von Wasserstoffperoxid bzw. durch Behandlung mit Ozon aufgeführt werden. Ein Verfahren, welches die kritische thermische Belastung des Rohproduktes aus Alkylglycosid und Alkohol vermeidet und somit nicht zur Dunkelfärbung führt, ist bislang nicht bekannt.

Die Reinigung von Zuckertensiden durch Behandlung mit überkritischem Kohlendioxid ist in der europäischen Patentanmeldung 0 404 226 beschrieben. Die der Extraktion unterworfenen Produkte enthalten Ester eines nichtreduzierenden Zuckers und eine oder mehrere Fettsäuren. Bei diesem Verfahren muß die Extraktion diskontinuierlich durchgeführt werden, da nach Abschluß der Abtrennung der Fettsäuren das Produkt in fester Phase vorliegt und der Trennprozeß daher zur Ausführung des Endproduktes unterbrochen werden muß.

Gegenstand der vorliegenden Erfindung ist ein Verfahren zur Herstellung von Alkylglycosiden durch Acetalisierung von Zuckern, insbesondere von Glucose, mit Alkoholen im Überschuß und nachfolgende Abtrennung des überschüssigen Alkohols durch Extraktion, dadurch gekennzeichnet, daß die Extraktion mit einem nahekritischen Extraktionsmittel, das sich im Bereich der reduzierten Temperatur von 0,8 bis 1,3, vorzugweise von 0,9 bis 1,2 befindet, bei Drükken, die so gewählt sind, daß die Dichte der nahekritischen Extraktionsmittel 180 kg/m³ bis 600 kg/m³ beträgt, durchgeführt wird, wobei als nahekritisches Extraktionsmittel ein gesättigter Kohlenwasserstoff, der 3 bis 8 Kohlenstoffatome aufweist oder ein Ether mit 2 bis 8 Kohlenstoffatomen verwendet wird.

Ein besonderer Vorteil des erfindungsgemäßen Verfahrens ist, daß unter den Bedingungen der Extraktion die Phase, welche die alkoholfreien Alkylglycoside enthält, im Gleichgewicht mit dem Extraktionsmittel flüssig ist und so Alkylglycoside hoher Reinheit kontinuierlich gewonnen werden können.

Insgesamt liefert das Verfahren dieser Erfindung folgende Vorteile:

a) die Alkohole können vollständig entfernt werden
b) die Extraktion kann kontinuierlich im Gegenstrom erfolgen
c) die Extraktionsmittel können bei niedrigen Temperaturen vollständig entfernt werden.

Unter reduzierter Temperatur (Tr) ist die Zahl zu verstehen, die sich durch Division der gemessenen Temperatur (in Kelvin) durch die kritische Temperatur (Tc) des Systems (in Kelvin) gemäß folgender Formel

$$Tr = \frac{T}{Tc}$$

ergibt.

Die Extraktion mit Kohlenwasserstoffen, insbesondere mit Propan, ist bevorzugt.

Den Extraktionsmitteln können bis zu 10 % niedermolekulare aliphatische Lösungsmittel und/oder Wasser zugesetzt werden. Als Beispiele für aliphatische Lösungsmittel sind zu nennen aliphatische Alkohole, wie Methanol, Ethanol, Isopropanol, Ketone, wie Aceton, Methylethylketon, Säuren, wie Ameisensäure, Essigsäure. Bevorzugt ist Ethanol.

Von den genannten Extraktionsmitteln werden Propan, Butan, Dimethylether und Ethylmethylether - insbesondere auch deren Gemische - wegen ihres niedrigen Siedepunktes unterhalb der Raumtemperatur besonders bevorzugt (Siedepunkt von Propan - 44,5 °C; von Butan - 0,5 °C; von Dimethylether - 24,9 °C). Sie sind als Extraktionsmittel für das Verfahren nach dieser Erfindung geeignet, wenn ihre Dichte mehr als 180 kg/m$^3$, vorzugsweise 200 bis 600 kg/m$^3$, beträgt. Besonders effektiv ist eine Arbeitsweise im Bereich einer reduzierten Temperatur des Extraktionsmittels von 0,8 bis 1,3 bei entsprechend erhöhten Drücken.

Als Alkylglycoside, welche durch das erfindungsgemäße Verfahren von nicht umgesetztem Alkohol befreit werden sollen, kommen insbesondere Verbindungen der allgemeinen Formel

$$R^1\text{-}R^2\text{-}O\text{-}G_a$$

in Betracht. Dabei haben die Reste folgende Bedeutung:

$R^1 =$  Kohlenwasserstoffrest mit 3 bis 21 C-Atomen,
$R^2 =$  $CH_2$, $(C_nH_{2n}O)_m$, wobei

    n  einen durchschnittlichen Zahlenwert von 2 bis 4, vorzugsweise 2 bis 3, und
    m  einen durchschnittlichen Zahlenwert von 1 bis 30, vorzugsweise 1 bis 10, hat,

G = von einer Aldose oder Ketose mit 5 oder 6 Kohlenstoffatomen abgeleiteter Rest, bevorzugt ist G von Glucose abgeleitet,
a = durchschnittlicher Zahlenwert von 1 bis 10, vorzugsweise 1 bis 6.

Hieraus ergibt sich, daß die abzutrennenden überschüssigen Alkohole der Formel $R^1$-$R^2$-OH entsprechen, wobei $R^1$ und $R^2$ die bereits angegebene Bedeutung haben, bevorzugt handelt es sich bei dem abzutrennenden Alkohol um einen Fettalkohol mit 8 bis 22 C-Atomen.

Die Abtrennung der Alkohole kann diskontinuierlich oder kontinuierlich im Gegenstrom-Verfahren erfolgen.

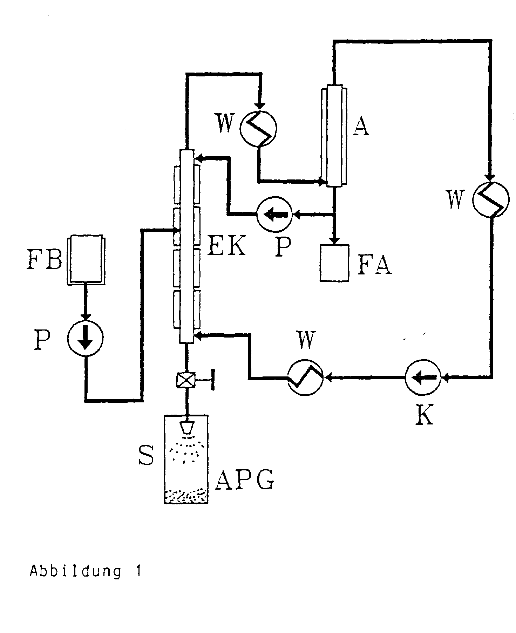

Die Erfindung wird durch die Abbildung 1 näher erläutert, die eine für die Durchführung des Verfahrens geeignete Extraktionsvorrichtung zeigt.

Wegen der wirtschaftlichen Vorteile wird das erfindungsgemäße Verfahren bevorzugt kontinuierlich im Gegenstrom durchgeführt. Die in Abbildung 1 schematisch dargestellte Apparatur besteht im wesentlichen aus einer Extraktionskolonne (Trennkolonne) und einer Abscheidekolonne (Regenerierkolonne).

Nach Vorwärmung beispielsweise auf etwa 130 °C wird das Gemisch aus z. B. Fettalkohol und Alkylglycosiden der Trennkolonne in der gewünschten Höhe, vorzugsweise etwa in der Mitte der Kolonne, zugepumpt. Das Extraktionsmittel wird zweckmäßig am Boden der Kolonne zugegeben und durchströmt die Extraktionskolonne von unten nach oben. Druck und Temperatur werden so eingestellt, daß die Mischung aus schwerflüchtigem Produkt und Extraktionsmittel in zwei flüssige Phasen zerfällt. Die Fettalkohole und eine geringe Menge Alkylglycoside lösen sich im Extraktionsmittel. Eine Mischung von Fettalkoholen, Extraktionsmitteln und einer geringen Menge an Alkylglycosiden verläßt die Extraktionskolonne am Kopf und wird der Regenerierkolonne zugeführt. Durch Erhöhung der Temperatur und/oder Verminderung des Druckes werden in der Regenerierkolonne Bedingungen eingestellt, unter denen das Extraktionsmittel als Dampf von so geringer Dichte vorliegt, daß die extrahierten Stoffe quantitativ ausfallen.

Das Extraktionsmittel verläßt die Regenerierkolonne am Kopf in dampfförmigem Zustand. Es wird in einem Wärmetauscher kondensiert und in die Extraktionskolonne zurückgeführt. Ein Teil des in der Regenerierkolonne ausgefallenen Produktes wird zweckmäßig als Rücklauf auf den Kopf der Extraktionskolonne zurückgepumpt. Der übrige Teil der extrahierten Fettalkohole wird am Sumpf der Regenerierkolonne abgezogen und auf Atmosphärendruck entspannt. Das dabei freiwerdende gasförmige Extraktionsmittel kann rekomprimiert, kondensiert oder verbrannt werden. Die extrahierten Fettalkohole können in den Reaktor für die Alkylglycosidherstellung zurückgeführt werden.

Infolge ihrer höheren Dichte fließen die Alkylglycoside in der Extraktionskolonne nach unten und werden dabei von den Fettalkoholen befreit. Die so erhaltenen reinen Alkylglycoside werden am Sumpf der Extraktionskolonne abgezogen. Durch Entspannung in einer Sprühvorrichtung auf Atmosphärendruck wird das Extraktionsmittel vollständig entfernt und das Raffinat in Form eines feinverteilten festen Pulvers gewonnen. Dabei wird die Temperatur vor der Versprühung so eingestellt, daß der Enthalpieinhalt der zu versprühenden Flüssigkeit zur Deckung der Verdampfungsenthalpie des Lösungsmittels ausreicht und die erreichte Endtemperatur des versprühten Gutes unterhalb der Erweichungstemperatur des entstehenden Pulvers liegt.

Um während des Extraktionsvorganges Zweiphasigkeit zu gewährleisten, ist die Extraktionskolonne zweckmäßig mit Einrichtungen zur Erzeugung eines Temperaturprofiles ausgerüstet. Wie bereits erwähnt, wird bevorzugt die Temperatur am Kopf der Extraktionskolonne 10 bis 80 °C, vorzugsweise 20 bis 50 °C, höher als am Kolonnensumpf eingestellt. Zur Erzeugung des Temperaturgefälles können beispielsweise Inline-Wärmetauscher dienen. Auf diese Weise wird dafür gesorgt, daß bei allen Mischungsverhältnissen zwischen Fettalkoholen und Alkylglycosiden zwei Phasen in der Kolonne existieren.

Die Erfindung wird in den folgenden Beispielen näher erläutert. Die Beispiele 1 bis 6 beschreiben die Auftrennung von Alkohol/ Alkylglycosid-Rohgemischen unter erfindungsgemäßen Bedingungen. Aus den Zusammensetzungen der jeweiligen Gas- und Flüssigphasen wird deutlich, daß in der Gasphase eine Anreicherung der Alkohole und in der Flüssigphase eine Anreicherung der Alkylglycoside stattfindet. Die Größe der sich daraus ergebenden Trennfaktoren reicht in jedem Fall aus, um in einem kontinuierlich durchgeführten mehrstufigen Prozeß zur Anreicherung von über 95 % der Alkylglycoside bzw. der Alkohole zu kommen. Die Abkürzung n. n. steht für nicht nachweisbar. Die Prozentangaben beziehen sich auf Gewichtsanteile.

Beispiel 1

Aus Fettalkohol, der im wesentlichen aus Dodecyl- und Tetradecylalkohol besteht, und wasserfreier Glucose wird in Analogie zur US-PS 3 839 318 ein Alkylglucosid hergestellt. Das Rohprodukt setzt sich nach Analyse (Silylierung und Hochtemperaturgaschromatographie) wie folgt zusammen:

| Decanol | 0,9 % | Monoglucosid | 14,7 % |
|---|---|---|---|
| Dodecanol | 58,7 % | Diglucosid | 3,4 % |
| Tetradecanol | 19,3 % | Triglucosid | 1,3 % |
| Hexadecanol | 1,0 % | Tetraglucosid | 0,4 % |
| | | Pentaglucosid | 0,2 % |
| | | | |
| Summe Fettalkohol | 79,9 % | Summe Alkylglucoside | 20,0 % |

In einen elektrisch beheizten 1-l-Schüttelautoklav werden 250 g des Rohproduktes gegeben und langsam erwärmt. Wenn die Temperatur 116 °C erreicht hat, wird Propan zugepumpt, bis sich ein Druck von 58 bar aufgebaut hat. Dazu werden etwa 170 g Propan benötigt. Durch Schütteln des Autoklaven wird für eine gute Durchmischung gesorgt. Ist der Autoklav in Ruhe, trennen sich rasch eine flüssige und eine gasförmige Phase. Mit Hilfe einer Kapillare mit einem Innendurchmesser von 0,3 mm werden Proben entnommen. Nach 30 Minuten ändert sich die Zusammensetzung der Phasen nicht mehr. Ihre Zusammensetzung wird durch Abdampfen des Propans und Hochtemperaturgaschromatographie bestimmt.

Zusammensetzung der Gasphase abzüglich Propan:

| Decanol | 1,2 % |
|---|---|
| Dodecanol | 74,4 % |
| Tetradecanol | 22,8 % |
| Hexadecanol | 1,2 % |
| | |
| Summe Fettalkohol | 99,6 % |

Zusammensetzung der Flüssigphase abzüglich Propan:

| Decanol | 0,7 % | Monoglucosid | 27,2 % |
|---|---|---|---|
| Dodecanol | 47,2 % | Diglucosid | 6,1 % |
| Tetradecanol | 16,0 % | Triglucosid | 2,2 % |
| Hexadecanol | 0,8 % | Tetraglucosid | 0,6 % |
| | | Pentaglucosid | 0,2 % |
| | | | |
| Summe Fettalkohol | 64,7 % | Summe Alkylglucoside | 36,3 % |

In der Gasphase befinden sich 10,7 % des Schwerflüchtigen, in der Flüssigphase 61,0 %. Die Gasphase enthält 89,3 % und die Flüssigphase 39,0 % Propan.

Beispiel 2

Der Versuch wird mit dem gleichen Rohstoff und analog zu Beispiel 1 durchgeführt. Im Unterschied zu Beispiel 1 wird Propan in den Autoklaven gepumpt, bis der Druck 68 bar beträgt, wozu etwa 210 g Propan benötigt werden. Nach Trennung der Phasen werden diese analysiert.

Zusammensetzung der Gasphase abzüglich Propan:

| Decanol | 1,0 % | Monoglucosid | 2,1 % |
|---|---|---|---|
| Dodecanol | 71,5 % | Diglucosid | 0,4 % |
| Tetradecanol | 23,6 % | Triglucosid | 0,1 % |
| Hexadecanol | 0,7 % | | |
| Summe Fettalkohol | 96,8 % | Summe Alkylglucoside | 2,6 % |

Zusammensetzung der Flüssigphase abzüglich Propan:

| Decanol | 0,4 % | Monoglucosid | 42,4 % |
|---|---|---|---|
| Dodecanol | 31,4 % | Diglucosid | 9,5 % |
| Tetradecanol | 10,6 % | Triglucosid | 3,4 % |
| Hexadecanol | 0,5 % | Tetraglucosid | 1,0 % |
| | | Pentaglucosid | 0,5 % |
| Summe Fettalkohol | 42,9 % | Summe Alkylglucoside | 56,8 % |

In der Gasphase befinden sich 26,5 % des Schwerflüchtigen, in der Flüssigphase 56,5 %. Die Gasphase enthält 73,5 % und die Flüssigphase 43,5 % Propan.

Beispiel 3

In einen elektrisch beheizten Schüttelautoklav mit 1 Liter Inhalt werden 250 g eines Ausgangsproduktes, dessen Zusammensetzung derjenigen des Rohproduktes des Beispiels 1 entspricht, eingefüllt und langsam erwärmt. Nachdem eine Temperatur von 190 °C erreicht ist, wird Dimethylether zugepumpt, bis sich ein Druck von 80 bar aufgebaut hat. Dazu werden etwa 250 g Dimethylether benötigt. Durch Schütteln des Autoklaven wird für eine gute Durchmischung gesorgt. Ist der Autoklav in Ruhe, scheiden sich rasch eine flüssige und eine gasförmige Phase ab. Mit Hilfe einer Kapillare von 0,3 mm Innendurchmesser werden Proben entnommen. Nach etwa 20 Minuten ändert sich die Zusammensetzung der Phasen nicht mehr. Ihre Zusammensetzung wird nach Abdampfen des Dimethylethers durch Hochtemperaturgaschromatographie bestimmt.

Zusammensetzung der gasförmigen Phase abzüglich Dimethylether:

| Decanol | 1,6 % | Monoglucosid *höhere Glucoside nicht nachweisbar* | 0,1 % |
|---|---|---|---|
| Dodecanol | 80,2 % | | |
| Tetradecanol | 17,7 % | | |
| Hexadecanol | 0,4 % | | |
| Summe Fettalkohol | 99,9 % | Summe Alkylglucoside | 0,1 % |

Zusammensetzung der Flüssigphase abzüglich Dimethylether:

| Decanol | 0,7 % | Monoglucosid | 20,4 % |
|---|---|---|---|
| Dodecanol | 54,7 % | Diglucosid | 4,4 % |

(fortgesetzt)

| Tetradecanol | 17,7 % | Triglucosid | 1,4 % |
|---|---|---|---|
| Hexadecanol | 0,6 % | Tetraglucosid | 0,6 % |
| | | Pentaglucosid | n. n. |
| | | | |
| Summe Fettalkohol | 73,2 % | Summe Alkylglucoside | 26,8 % |

Die Gasphase enthält 92,5 % Dimethylether und 7,5 % an Fettalkoholen und Alkylglucosiden. Die Flüssigphase enthält 38 % Dimethylether.

Beispiel 4

Aus Fettalkohol, der im wesentlichen aus Hexadecyl- und Octadecylalkohol besteht, und wasserfreier Glucose wird in Analogie zur US-PS 3 839 318 ein Alkylglucosid hergestellt, das im folgenden verkürzt als Stearylglucosid bezeichnet wird. Das Rohprodukt entspricht der folgenden Zusammensetzung:

| Tetradecanol | < 0,1 % | Monoglucosid | 26,9 % |
|---|---|---|---|
| Hexadecanol | 15,6 % | Diglucosid | 4,6 % |
| Octadecanol | 50,8 % | Triglucosid | 2,0 % |
| Eicosanol | < 0,1 % | Tetraglucosid | < 0,1 % |
| | | Pentaglucosid | < 0,1 % |
| | | | |
| Summe Fettalkohol | 66,4 % | Summe Alkylglucoside | 33,6 % |

In einen elektrisch beheizten Schüttelautoklaven mit 1 Liter Inhalt werden 250 g des Stearylglucosides eingefüllt und langsam erwärmt. Nachdem eine Temperatur von 122 °C erreicht ist, wird Propan bis zu einem Druck von 70 bar zugepumpt. Dazu werden 250 g Propan benötigt. Durch Schütteln des Autoklaven wird für eine gute Durchmischung gesorgt. Bei Ruhestellung des Autoklaven scheiden sich rasch eine flüssige und eine gasförmige Phase ab. Mit Hilfe einer Kapillare von 0,3 mm Innendurchmesser werden Proben entnommen. Nach etwa 20 Minuten ändert sich die Zusammensetzung der Phasen nicht mehr. Nach Abdampfen des Propans wird die Zusammensetzung des zurückgebliebenen schwerflüchtigen Anteils durch Hochtemperaturgaschromatographie bestimmt.

Zusammensetzung der gasförmigen Phase abzüglich Propan:

| Dodecanol | < 0,1 % | Monoglucosid | 10,9 % |
|---|---|---|---|
| Tetradecanol | 0,1 % | Diglucosid | < 0,1 % |
| Hexadecanol | 20,8 % | Triglucosid | n. n. |
| Octadecanol | 68,2 % | Tetraglucosid | n. n. |
| Eicosanol | n. n. | Pentaglucosid | n. n. |
| | | | |
| Summe Fettalkohole | 89,1 % | Summe Alkylglucoside | 10,9 % |

Zusammensetzung der Flüssigphase abzüglich Propan:

| Dodecanol | n. n. | Monoglucosid | 62,0 % |
|---|---|---|---|
| Tetradecanol | 0,2 % | Diglucosid | 19,0 % |
| Hexadecanol | 4,5 % | Triglucosid | < 0,1 % |
| Octadecanol | 14,1 % | Tetraglucosid | n. n. |
| Eicosanol | n. n. | Pentaglucosid | n. n. |
| | | | |
| Summe Fettalkohole | 18,9 % | Summe Alkylglucoside | 81,0 % |

Die Gasphase enthält 81 % Propan; die Flüssigphase 23 % Propan.

## Beispiel 5

In einem Dreihalskolben mit Rückflußkühler und Wasserabscheider werden unter Stickstoffdurchleitung 729 g (5,9 Mol) n-Butanol, 5,1 g p-Toluolsulfonsäuremonohydrat gegeben und auf 120 °C erwärmt. Bei dieser Temperatur werden in Portionen zu ca. 5 g 300 g (1,66 Mol) wasserfreie Glucose im Verlaufe 1 Stunde gegeben. Während dieser Phase scheidet sich bereits Wasser ab, ein Zeichen für den Fortschritt der Glucosidbildung. Diese Reaktion wird bei 120 °C fortgesetzt, bis sich kein weiteres Wasser mehr abscheidet, was nach etwa einer weiteren Stunden der Fall ist. Die Reaktionsmischung wird auf 65 °C abgekühlt, mit 36,6 g 5 %iger ethanolischer KOH-Lösung alkalisch gestellt (pH-Wert einer 30 %igen Lösung in Wasser/Ethanol 1 : 2 ca. 8) und filtriert.

150 g des Rohproduktes, das aus 60,6 Gew.-% Butanol und 39,4 Gew.-% Butylglucosid besteht, werden in einen elektrisch beheizten 1-Liter-Schüttelautoklav gefüllt und langsam erwärmt. Nachdem eine Temperatur von 91 °C erreicht ist, wird Propan bis zu einem Druck von 35 bar zugepumpt. Dazu werden ca. 150 g Propan benötigt. Durch Schütteln des Autoklaven wird für eine gute Durchmischung gesorgt. Bei Ruhestellung des Autoklaven scheiden sich rasch eine flüssige und eine gasförmige Phase ab. Mit Hilfe einer Kapillare von 0,3 mm Innendurchmesser werden Proben entnommen. Nach etwa 20 Minuten wird keine Änderung der Zusammensetzung der Phasen mehr festgestellt. Nach Abdampfen des Propans wird die Zusammensetzung des schwerflüchtigen Anteils durch Hochtemperaturgaschromatographie bestimmt.

Die Flüssigphase besteht nach Abzug des Propans aus 23,2 Gew.-% Butanol und 76,8 Gew.-% Butylglucosiden; die Gasphase aus 83,2 Gew.-% Butanol und 16,8 Gew.-% Butylglucosiden. Die Gasphase enthält 69,3 Gew.-% Propan und 30,7 Gew.-% schwerflüchtige Anteile. Die Flüssigphase enthält 9,4 Gew.-% Propan.

## Beispiel 6

In einen elektrisch beheizten Schüttelautoklav mit 1 Liter Inhalt werden 250 g eines $C_{12}/C_{14}$-Fettalkoholgelucosides der folgenden Zusammensetzung:

| | | | |
|---|---|---|---|
| Dodecanol | 1,9 % | Monoglucosid | 70,0 % |
| Tetradecanol | 3,0 % | Diglucosid | 15,5 % |
| Hexadecanol | 0,4 % | Triglucosid | 5,3 % |
| | | Tetraglucosid | 1,7 % |
| | | Pentaglucosid | 0,7 % |
| Summe Fettalkohole | 5,3 % | Summe Alkylglucoside | 93,2 % |

und 30 g Ethanol eingefüllt und langsam erwärmt.

Das eingesetzte $C_{12}/C_{14}$-Fettalkoholglucosid ist, wie in Beispiel 1 beschrieben, hergestellt und anschließend durch Destillation vom größten Teil des Fettalkohols befreit worden.

Nachdem eine Temperatur von 105 °C erreicht ist, wird Butan bis zu einem Druck von 23 bar zugepumpt. Durch Schütteln des Autoklaven wird für eine gute Durchmischung gesorgt. Ist der Autoklav in Ruhe, scheiden sich rasch eine flüssige und eine gasförmige Phase ab. Mit Hilfe einer Kapillare von 0,3 mm Innendurchmesser werden Proben aus beiden Phasen entnommen und analysiert. Die gasförmige Phase enthält 5,8 Gew.-% an Fettalkoholen und Glucosiden. Abzüglich des Butan- und Ethanolgehaltes besteht das in der gasförmigen Phase gelöste Produkt zu 19,2 Gew.-% aus Fettalkoholen und zu 80,6 Gew.-% aus Alkylglucosiden. Die flüssige Phase enthält abzüglich des Butan- und Ethanolgehaltes 2,7 Gew.-% Fettalkohole und 96,8 Gew.-% Alkylglucoside.

## Beispiel 7

250 g eines Gemisches aus Fettalkoholen und $C_{12}/C_{14}$-Fettalkoholglucosiden, dessen Zusammensetzung derjenigen des Ausgangsproduktes im Beispiel 6 entspricht, und 60 g Ethanol werden in einen elektrisch beheizten Schüttelautoklaven mit 1 Liter Inhalt eingefüllt und langsam auf 85 °C erwärmt. Danach wird Propan bis zu einem Druck von 40 bar zugepumpt. Durch Schütteln des Autoklaven wird für eine gute Durchmischung gesorgt. Ist der Autoklav in Ruhe, scheiden sich rasch eine gasförmige und eine flüssige Phase ab. Mit Hilfe einer Kapillare von 0,3 mm Innendurchmesser werden Proben aus beiden Phasen entnommen. Nach etwa 20 Minuten ändert sich ihre Zusammensetzung nicht mehr. Nach Abdampfen des Propans werden die Proben durch Hochtemperaturgaschromatographie analysiert. Die Beladung der Gasphase mit Fettalkoholen und Alkylglucosiden beträgt 7,9 Gew.-%. Abzüglich des Propan- und Ethanolgehaltes besteht das in der gasförmigen Phase gelöste Produkt aus 13,7 Gew.-% Fettalkoholen und 86,0 Gew.-% Alkylglucosiden. Die flüssige Phase enthält abzüglich Propan und Ethanol 2,9 Gew.-% Fettalkohole und 96,6

Gew.-% Alkylglucoside.

<u>Beispiel 8</u>

Die Alkohole werden aus dem Produktgemisch aus überschüssigen Alkoholen und Alkylglucosiden, wie es in Beispiel 1 als Rohprodukt beschrieben ist, in einer Anlage nach Figur 1 abgetrennt. Das Produktgemisch wird etwa in der Mitte der Extraktionskolonne (Trennkolonne) zugepumpt. Die Kolonne ist mit einer Sulzerpackung CY und mit Heizelementen zur Regelung der Temperatur in den verschiedenen Kolonnenabschnitten versehen. Die Zahl der theoretischen Trennstufen der Kolonne beträgt etwa 4. Als Extraktionsmittel dient Propan bei einem Druck von ca. 70 bar. Die Temperatur in der Extraktionskolonne nimmt vom Sumpf der Kolonne von 120 °C bis zum Kopf auf 140 °C linear zu.

Durch die Erhöhung der Temperatur zum Kopf der Extraktionskolonne wird die Löslichkeit der Alkylglucoside im dichten Propan soweit herabgesetzt, daß die Verluste an Alkylglucosiden in Verbindung mit dem Austrag der Fettalkohole gering sind (weniger als 3 %). Das mit den Fettalkoholen beladene Propan wird der Abscheidekolonne A zugeführt. Durch Druckabsenkung auf ca. 30 bar in der Abscheidekolonne wird die Dichte des Propans bei 140 °C soweit verringert, daß die gelösten Fettalkohole quantitativ ausfallen. Das Fettalkoholgemisch fällt als propanhaltige Flüssigkeit an. Ein Teil der im Sumpf der Abscheidekolonne angefallenen flüssigen Fettalkohole wird auf den Kopf der Extraktionskolonne als Rücklauf zurückgepumpt.

Das gasförmige überkritische Propan verläßt den Abscheider am Kopf und wird in einem nachgeschalteten Wärmetauscher abgekühlt und kondensiert. Anschließend wird das flüssige Propan auf den Extraktionsdruck von 70 bar rekomprimiert und über einen Wärmetauscher, in dem es auf die Temperatur des Sumpfes der Extraktionskolonne erwärmt wird, in die Extraktionskolonne zurückgeführt.

Das zu trennende Gemisch fließt in der Extraktionskolonne im Gegenstrom zum Propan nach unten und kommt dabei mit immer reinerem Propan in Kontakt. Durch die Gegenstromführung wird eine weitgehende Entfernung der Fettalkohole erreicht. Das Sumpfprodukt aus der Extraktionskolonne wird über eine Sprühdüse auf Atmosphärendruck entspannt. Die Entspannung erfolgt in einen Sprühturm hinein. Dabei verdampft das gelöste Propan vollständig und kühlt die von Fettalkoholen befreiten Alkylglucoside, die als feinverteiltes Pulver erhalten werden, auf Temperaturen unter 60 °C ab.

Nachstehend sind die Analysen von Ausgangsprodukt, Raffinat und Extrakt gegenübergestellt. Als Raffinat wird das Sumpfprodukt (glucosidreich) und als Extrakt das Kopfprodukt (fettalkoholreich) der Abscheidekolonne bezeichnet.

| | | Ausgangsprodukt Gew.-% | Raffinat Gew.-% | Extrakt Gew.-% |
|---|---|---|---|---|
| **Fettalkohol**: | C 10 | 0,8 | < 0,1 | 0,9 |
| | C 12 | 60,1 | 0,1 | 74,0 |
| | C 14 | 20,0 | < 0,1 | 24,0 |
| | C 16 | 1,0 | < 0,1 | 0,9 |
| | C 18 | n. n. | < 0,1 | < 0,1 |
| | C 20 | n. n. | n. n. | n. n. |
| ***Summe Fettalkohol*** | | ***81,9*** | ***0,1*** | ***99,8*** |
| **Glucosid:** | Mono- | 13,4 | 72,7 | 0,19 |
| | Di- | 3,2 | 19,0 | 0,01 |
| | Tri- | 1,1 | 6,8 | n. n. |
| | Tetra- | 0,4 | 1,4 | n. n. |
| | Penta- | < 0,1 | < 0,1 | n. n. |
| ***Summe Glucoside*** | | ***18,1*** | ***99,9*** | ***0,2*** |

Zu Abbildung 1:

Schematisches Verfahrensfließbild zur Entfernung von Alkohol aus Gemischen von Alkoholen und Alkylglycosiden mit Hilfe der kontinuierlichen Gegenstromextraktion

A: Abscheidekolonne
EK: Extraktionskolonne
FB: Feedbehälter
P: Pumpe
W: Wärmeaustauscher
APG: Alkylglycosid
FA: Alkohol
K: Kreisgaskompressor
S: Sprühturm

**Patentansprüche**

1. Verfahren zur Herstellung von Alkylglycosiden durch Acetalisierung von Zuckern, insbesondere von Glucose, mit Alkoholen im Überschuß und nachfolgende Abtrennung des überschüssigen Alkohols durch Extraktion, dadurch gekennzeichnet, daß die Extraktion mit einem nahekritischen Extraktionsmittel, das sich im Bereich der reduzierten Temperatur von 0,8 bis 1,3, vorzugsweise von 0,9 bis 1,2 befindet, bei Drucken, die so gewählt sind, daß die Dichte der nahekritischen Extraktionsmittel 180 kg/m$^3$ bis 600 kg/m$^3$ beträgt, durchgeführt wird, wobei als nahekritisches Extraktionsmittel ein gesättigter Kohlenwasserstoff, der 3 bis 8 Kohlenstoffatome aufweist oder ein Ether mit 2 bis 8 Kohlenstoffatomen verwendet wird.

2. Verfahren nach Anspruch 1, dadurch gekennzeichnet, daß als nahekritisches Extraktionsmittel Dimethylether verwendet wird.

3. Verfahren nach Anspruch 1, dadurch gekennzeichnet, daß als nahekritisches Extraktionsmittel Ethylmethylether verwendet wird.

4. Verfahren nach Anspruch 1, dadurch gekennzeichnet, daß als nahekritisches Extraktionsmittel Propan oder Butan oder deren Mischungen verwendet wird.

5. Verfahren nach Anspruch 1, dadurch gekennzeichnet, daß die Siedetemperatur der verwendeten Extraktionsmittel unterhalb der Raumtemperatur liegt.

6. Verfahren nach Anspruch 1 bis 5, dadurch gekennzeichnet, daß die Extraktion bei einem Druck durchgeführt wird, der bis etwa 80 bar, vorzugsweise 10 bis 60 bar höher ist als der Dampfdruck des Extraktionsmittels über der Lösung.

7. Verfahren nach Anspruch 1 bis 6, dadurch gekennzeichnet, daß die Extraktion mehrstufig im Gegenstrom durchgeführt wird.

8. Verfahren nach Anspruch 1 bis 7, dadurch gekennzeichnet, daß das Raffinat, welches am Boden der Extraktionskolonne (Trennkolonne) abgezogen wird, über eine Sprühdüse entspannt und das von Alkoholen befreite Produkt in Form eines Pulvers erhalten wird.

9. Verfahren nach Anspruch 1 bis 8, dadurch gekennzeichnet, daß Gemische aus Ethern und Kohlenwasserstoffen eingesetzt werden.

10. Verfahren nach einem oder mehreren der vorhergehenden Ansprüche, dadurch gekennzeichnet, daß den als Extraktionsmittel benutzten Kohlenwasserstoffen oder Ethern niedermolekulare aliphatische Lösungsmittel und/oder Wasser bis zu 10 Gew.-% zugesetzt werden.

**Claims**

1. Process for preparing alkyl glycosides by acetalization of sugars, in particular glucose, with alcohols in excess and subsequent removal of the excess alcohol by extraction, characterized in that the extraction is carried out using a near-critical extraction medium, which is in the range of reduced temperature of 0.8 to 1.3, preferably 0.9 to 1.2, at pressures which are chosen such that the density of the near-critical extraction medium is 180 kg/m$^3$ to 600 kg/

m$^3$, the near-critical extraction medium used being a saturated hydrocarbon which has 3 to 8 carbon atoms or an ether having 2 to 8 carbon atoms.

2. Process according to Claim 1, characterized in that the near-critical extraction medium used is dimethyl ether.

3. Process according to Claim 1, characterized in that the near-critical extraction medium used is ethyl methyl ether.

4. Process according to Claim 1, characterized in that the near-critical extraction medium used is propane or butane or mixtures thereof.

5. Process according to Claim 1, characterized in that the boiling temperature of the extraction medium used is below room temperature.

6. Process according to Claims 1 to 5, characterized in that the extraction is carried out at a pressure which is up to about 80 bar, preferably 10 to 60 bar, higher than the vapour pressure of the extraction medium above the solution.

7. Process according to Claims 1 to 6, characterized in that the extraction is carried out in a plurality of stages in counter-current.

8. Process according to Claims 1 to 7, characterized in that the raffinate which is taken off at the bottom of the extraction column (separation column) is expanded via a spray nozzle and the product which is freed from alcohols is obtained in the form of a powder.

9. Process according to Claims 1 to 8, characterized in that mixtures of ethers and hydrocarbons are used.

10. Process according to one or more of the preceding Claims, characterized in that low-molecular-weight aliphatic solvents and/or water are added up to 10% by weight to the hydrocarbons or ethers used as extraction medium.


**Revendications**

1. Procédé pour la préparation d'alcoylglycosides par acétalisation de glucides, en particulier du glucose, avec des alcools en excès puis par séparation de l'alcool en excès par extraction, caractérisé en ce que l'extraction est réalisée avec un solvant d'extraction quasi critique, qui se situe dans le domaine de la température réduite de 0,8 à 1,3, de préférence de 0,9 à 1,2, sous des pressions qui sont choisies de telle sorte que la densité du solvant d'extraction quasi critique soit de 180 kg/m$^3$ à 600 kg/m$^3$, ce solvant d'extraction quasi critique utilisé étant un hydrocarbure saturé qui présente 3 à 8 atomes de carbone ou un éther ayant 2 à 8 atomes de carbone.

2. Procédé suivant la revendication 1, caractérisé en ce que le solvant d'extraction quasi critique utilisé est le diméthyléther.

3. Procédé suivant la revendication 1, caractérisé en ce que le solvant d'extraction quasi critique utilisé est l'éthylméthyléther.

4. Procédé suivant la revendication 1, caractérisé en ce que le solvant d'extraction quasi critique utilisé est le propane ou le butane ou leurs mélanges.

5. Procédé suivant la revendication 1, caractérisé en ce que le point d'ébullition du solvant d'extraction utilisé se situe en dessous de la température ambiante.

6. Procédé suivant l'une quelconque des revendications 1 à 5, caractérisé en ce que l'extraction est réalisée sous une pression qui est supérieure d'environ 80 bars, de préférence de 10 à 60 bars, à la pression de vapeur du solvant d'extraction au-dessus de la solution.

7. Procédé suivant l'une quelconque des revendications 1 à 6, caractérisé en ce que l'extraction est réalisée en plusieurs étapes à contre-courant.

8. Procédé suivant l'une quelconque des revendications 1 à 7, caractérisé en ce que le raffinat, qui est soutiré à la base de la colonne d'extraction (colonne de séparation), est détendu par un atomiseur et en ce que le produit débarrassé des alcools est obtenu sous la forme d'une poudre.

9. Procédé suivant l'une quelconque des revendications 1 à 8, caractérisé en ce que l'on utilise un mélange d'éthers et d'hydrocarbures.

10. Procédé suivant une ou plusieurs des revendications précédentes, caractérisé en ce que l'on utilise, comme hydrocarbures ou comme éthers utilisés comme solvant d'extraction, un solvant aliphatique de bas poids moléculaire et/ou de l'eau jusqu'à 10% en poids.

Abbildung 1